(19) Europäisches Patentamt
European Patent Office
Office européen des brevets

(11) Publication number: **0 402 062 A2**

# (12) EUROPEAN PATENT APPLICATION

(21) Application number: 90306006.9

(51) Int. Cl.5: C08F 8/32, C08F 8/44, A61K 31/74

(22) Date of filing: 01.06.90

(30) Priority: 05.06.89 GB 8912903

(43) Date of publication of application: 12.12.90 Bulletin 90/50

(84) Designated Contracting States: CH DE FR GB IT LI NL

(71) Applicant: SMITH KLINE & FRENCH LABORATORIES LIMITED
Mundells
Welwyn Garden City Hertfordshire, AL7 1EY(GB)

(72) Inventor: Jaxa-Chamiec, Albert Andrzej
22 Beacon Way
Rickmansworth, Hertfordshire, WD3 2PE(GB)
Inventor: Hickey, Deirdre Mary Bernadette
Smith Kline & French Research Limited, The Frythe
Welwyn, Hertfordshire, AL6 9AR(GB)
Inventor: Shah, Virendra Premchand
Smith Kline & French Research Limited, The Frythe
Welwyn, Hertfordshire, AL6 9AR(GB)

(74) Representative: Giddings, Peter John, Dr. et al
SmithKline Beecham, Corporate Patents, Mundells
Welwyn Garden City, Hertfordshire AL7 1EY(GB)

## (54) Compounds.

(57) Polymers of structure:

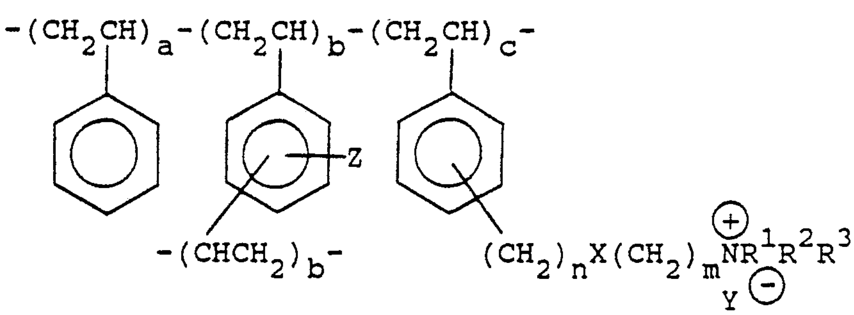

in which,
n is 0 or 1;
m is 1 to 20;
Z is H or a group $(CH_2)_nX(CH_2)_m\overset{\oplus}{N}R^1R^2R^3Y^{\ominus}$,
X is 0, S, $SO_2$, amido or sulphonamido;
$R^1$ and $R^2$ are the same or different and are each $C_{1-4}$alkyl;
$R^3$ is $C_{1-20}$alkyl; or $R^1$ to $R^3$ together with the nitrogen atom to which they are attached form a ring, optionally containing one or more further heteroatoms and optionally substituted by a $C_{1-4}$alkyl group;
a, b and c are numbers which indicate the relative molar percentages of the units present in a random distribution in said polymer, (b) being from about 0.5 to about 10 molar percent, and (c) being from about 30 to about 99 molar percent; and

EP 0 402 062 A2

$Y^{\ominus}$ is a physiologically acceptable counter ion,
processes for their preparation, pharmaceutical compositions containing them and their use in therapy as cholesterol lowering agents.

# COMPOUNDS

The present invention relates to novel polystyrene anion exchange polymers, processes for their preparation, intermediates useful in their preparation, pharmaceutical compositions containing them and their use in the lowering of plasma cholesterol levels in humans.

Coronary Heart Disease (CHD) is one of the most serious health problems of contemporary society. Worldwide epidemiological studies have shown that the incidence of CHD is related to a number of independent risk factors, in particular, for example, high concentrations of serum cholesterol (hypercholesterolaemia). Such adverse factors lead to atherosclerosis, and ultimately, in severe cases, intermittent claudication, cerebrovascular insufficiency, thrombosis and cardiac arrest.

It is known that ion exchange polymers, in particular polystyrene polymers can be used as sequestering agents to bind non-absorbed bile acids and salts in the intestinal tract, forming complexes which are then excreted in the faeces. This sequestering leads to a decrease in the amount of bile acids returning to the liver via enterohepatic circulation. The synthesis of replacement bile acids from hepatic cholesterol depletes hepatic cholesterol, regulates hepatic LDL receptors and consequently reduces plasma cholesterol levels. Such sequestering polymers have been recognised as useful for the treatment of hypercholesterolaemia, and it is now proven that reducing serum cholesterol with bile acid sequestrants has a beneficial effect on protecting against the occurrence of coronary heart disease.

The polystyrene polymers known in the art to have such sequestering activity are, in general, those bearing a di- or tri-loweralkyl ammonium group, such as a trimethylammonium group. For example, GB 1286949 discloses a series of macroporous polystyrene polymers having 5-20% cross-link, and GB 1579490 discloses a series of microporous polymers having 8-20% cross-link. In addition, GB 2026501 discloses a series of, *inter alia*, polystyrene polymers which are said to have particular water absorption capacities, i.e. 69-73% *by weight* of polymer weight. In each of the foregoing, the polystyrene polymers bear di- or tri-loweralkyl ammonium groups, in particular a trimethylammonium group.

One particular agent based on a polystyrene polymer which is currently used to lower serum cholesterol levels in humans by binding bile acids in the intestinal tract is cholestyramine (GB 929391). Cholestyramine is a cross-linked anion exchange polystyrene polymer bearing an ionised trimethylammonium groups bound to the polymer backbone. However, the use of this agent is associated with a number of undesirable side-effects, for example, it is unpalatable and must be taken in large doses (up to 36 g per day) and causes, in some cases, bloating, constipation and other gut side-effects. In addition, its ability to bind bile acids is inefficient with respect to the amounts of polymer which it is necessary to use.

The present invention therefore provides in a first aspect, polystyrene polymers of structure (I) :

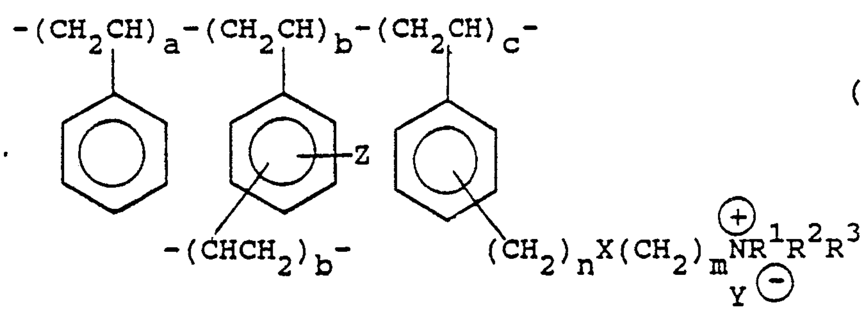

(I)

in which,

n is 0 or 1;

m is 1 to 20;

Z is H or a group $(CH_2)_nX(CH_2)_m\overset{\oplus}{N}R^1R^2R^3Y^\ominus$;

X is O, S, $SO_2$, amido or sulphonamido;

$R^1$ and $R^2$ are the same or different and are each $C_{1-4}$alkyl;

$R^3$ is $C_{1-20}$alkyl; or $R^1$ to $R^3$ together with the nitrogen atom to which they are attached form a ring, optionally containing one or more further heteroatoms and optionally substituted by a $C_{1-4}$alkyl group;

a, b and c are numbers which indicate the relative molar percentages of the units present in a random distribution in said polymer, (b) being from about 0.5 to about 10 molar percent, and (c) being from about 30 to about 99 molar percent; and

$Y^{\ominus}$ is a physiologically acceptable counter ion.

Suitably n is 0 or 1; preferably n is 1. Suitably m is 1 to 20; preferably m is 1 to 12.

Suitably X is O, S, $SO_2$, amido or sulphonamido; preferably X is O, S, amido or sulphonamido; most preferably X is oxygen or sulphur.

Suitably $R^1$, $R^2$ and $R^3$ together with the nitrogen atom to which they are attached form a ring, optionally containing one or more further heteroatoms and optionally substituted by a $C_{1-4}$alkyl group. More suitably $R^1$ and $R^2$ are the same or different and are each $C_{1-4}$alkyl and $R^3$ is $C_{1-20}$alkyl; preferably $R^1$ and $R^2$ are the same and are both methyl, and $R^3$ is a $C_{1-8}$alkyl group, in particular a methyl group.

Suitably (b) is from about 0.5 to about 10 molar percent of said polymer, preferably (b) is from about 1 to about 8 molar percent of said polymer; most preferably from about 1 to about 4 molar percent.

Suitably $Y^{\ominus}$ is a physiologically acceptable counter ion such as a sulphate, bicarbonate, carbonate, formate, acetate, sulphonate, propionate, malonate, succinate, malate, tartrate, citrate, maleate, fumarate, ascorbate, glucuronate, phosphate, or halide, or the anion of an amino acid such as aspartic or glutamic acid. More suitably $Y^{\ominus}$ is a phosphate, sulphate or a halide ion; preferably a halide ion, in particular chloride.

The term amido used herein above shall be taken to include the following groups: -NRCO- and -CONR in which R is hydrogen or $C_{1-4}$alkyl.

The term sulphonamido used herein above shall be taken to include the following groups: $-SO_2NH-$ and $-NHSO_2-$.

The polystyrene polymers of the present invention are also characterised by their total exchange capacity i.e. the theoretical maximum capacity of the polymer if each counter ion were to be exchanged with bile acid. In this specification the total exchange capacity is defined in terms of the number of milliequivalents of counter ion per gram of dry weight of polymer.

Suitable total exchange capacities are in the range of, for example where the counter ion $Y^{\ominus}$ is a halide ion such as chlorine, from 1.5 to 3.5 meq $Cl^-$ per gram of polymer. Preferred within this range are polymers having a total exchange capacity of between 1.5 and 2.5 meq $Cl^-$ gram of polymer.

In addition, it is to be noted that the approximate molar percentages (a), (b) and (c) are calculated from the monomer mixture or, in some instances (c) from microanalytical data.

It is to be noted that the term 'bile acid' when used herein shall be taken to include bile acids, bile salts and conjugates thereof.

The polystyrene polymers of the present invention can be prepared by processes analogous to those known in the art. The present invention therefore provides, in a further aspect, a process for preparing the polystyrene polymers of structure (I) which comprises :

(a) reaction of a polymer of structure (II)

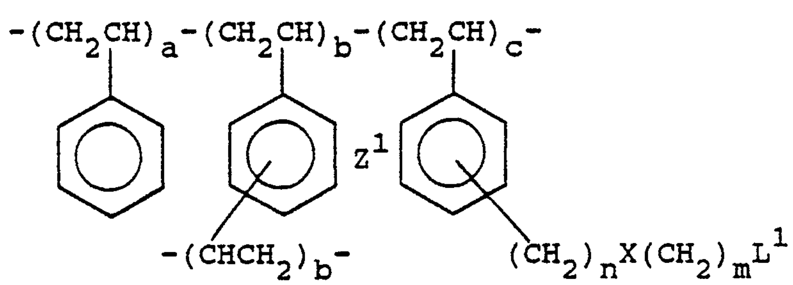

(II)

in which $Z^1$ is hydrogen or $(CH_2)_nX(CH_2)_mL^1$ and X, b, c, n and m are as described for structure (I) and $L^1$ is a group displaceable by an amine, with an amine of structure $R^1R^2R^3N$ (III) in which $R^1$ to $R^3$ are as described for structure (I); or

(b) for compounds of structure (I) in which $R^1$ and $R^2$ are the same or different and are each $C_{1-4}$alkyl and $R^3$ is $C_{1-20}$alkyl; reaction of a compound of structure (IV)

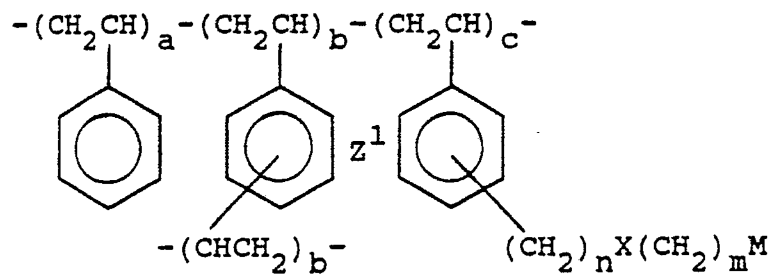

(IV)

in which $Z^1$ is hydrogen or $(CH_2)_nX(CH_2)_mM$, X, a, b, c, n and m are as described for structure (I) and M is $NR^1R^2$ or $NR^2R^3$ in which $R^1$ to $R^3$ are as described for structure (I), with a compound of structure $R^4L^2$ (V) in which $R^4$ is a $C_{1-4}$alkyl group when M is $NR^1R^3$ or a $C_{1-20}$ alkyl group when M is $NR^1R^2$, and $L_2$ is a group displaceable by an amine.

Suitable leaving groups displaceable by an amine $L^1$ and $L^2$ include for example, halogen, in particular chlorine and bromine, others will be apparent to those skilled in the art, and include for example mesylates and tosylates.

The reaction between a polymer of structure (II) and an amine of structure (III) can be carried out in a suitable solvent at elevated temperature. Suitable solvents include for example, a $C_{1-4}$alkanol, N-methylpyrrolidone, sulpholane, dimethylformamide, nitromethane or tetrahydrofuran. Preferably the reaction is carried out in N-methylpyrrolidone at a temperature of between about 50° and 80° for up to 24 hours or until the reaction is complete.

The reaction between a polymer of structure (IV) and a compound of structure (V) can be carried out in a suitable inert solvent such as a $C_{1-4}$alkanol, nitromethane, sulpholane, N-methylpyrrolidone, dimethylformamide or tetrahydrofuran at elevated temperature.

The intermediate polymers of structure (II) can be prepared by methods analogous to those known in the art, as detailed in the specific examples hereinafter. For example:

° Compounds of structure (11) in which n is 0, X is NHCO and L' is bromine can be prepared from compounds of structure (IIA)

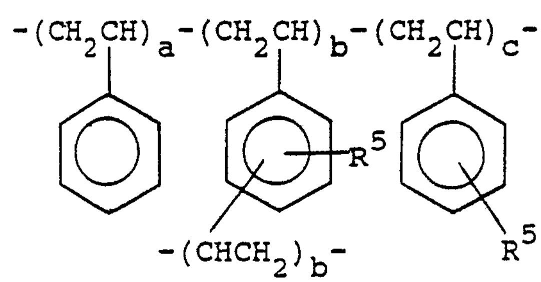

(IIA)

in which a, b and c are as described for structure (II) and $R^5$ is $NH_2$ by reaction with a suitable compound of structure $L^3\overset{O}{\overset{\|}{C}}(CH_2)_nBr$, in which $L^3$ is a suitable leaving group, for example, halogen, in particular chlorine. Compounds of structure (IIA) in which $R^5$ is $NH_2$ can be prepared from the corresponding compounds in which $R^5$ is $NO_2$ by reduction with, for example $SnCl_2$. Compounds of structure (IIA) in which $R^5$ is $NO_2$ can themselves be prepared by nitration of polystyrene with, for example $NH_4NO_3$ in a suitable solvent such as trifluoroacetic anhydride. Polystyrene is commercially available.

° Compounds of structure (II) in which n is O, X is -NMeCO- and $L^1$ is bromine, are prepared by a similar sequence of reactions from compounds of structure (IIA) in which $R^5$ is NHMe. Such compounds of structure (IIA) can be prepared from compounds of structure (IIA) in which $R^5$ is $NH_2$, by reaction with, for example, acetic anhydride in formic acid followed by $BH_3$ in dimethylsulphide;

° Compounds of structure (II) in which n is 1, X is NHCO and $L^2$ is bromine can be prepared from the corresponding compounds of structure (IIA) in which $R^2$ is $CH_2NH_2$ using reactions analogous to those described above. The compounds of structure (IIA) in which $R^5$ is $CH_2NH_2$ can be prepared from chloromethylated polystyrene (IIA, $R^5$ = $CH_2Cl$, commercially available), by reaction with for example potassium phthalmide in a suitable solvent such as dimethylformamide, followed by conversion of the

phthalamide group to an amino group by reaction with hydrazine and a suitable base such as sodium hydroxide.

° Compounds of structure (II) in which n is O, X is S and $L^1$ is bromine, can be prepared from compounds of structure (II) in which n is O, X is S and $L^1$ is hydroxy by reaction with, for example, carbon tetrabromide in triphenylphosphine. Compounds of structure (II) in which n is O, X is S and $L^1$ is hydroxy can themselves be prepared from compounds of structure (IIA) in which $R^5$ is SH, by reaction with for example $Br(CH_2)_nOH$ in the presence of a base such as sodium hydroxide. Compounds of structure (IIA) in which $R^5$ is SH can be prepared from compounds of structure (IIA) in which $R^5$ is bromine by reaction with, for example, n-butyl lithium and sulphur.

° Compounds of structure (II) in which n is O or 1, X is $NHSO_2$, and $L^1$ is chlorine can be prepared from the corresponding compounds of structure (IIA) in which $R^5$ is $NH_2$ or $CH_2NH_2$ by reaction with a suitable compound of structure $ClSO_2(CH_2)_nCl$.

° Compounds of structure (IV) in which n is O, X is $SO_2NH$ and M is $NR^1R^2$ can be prepared from compounds of structure (IIA) in which $R^5$ is $SO_2Cl$, by reaction with, for example, a compound of structure $NH_2(CH_2)_mNR^1R^2$ in a suitable solvent such as dichloromethane. The compounds of structure (IIA) in which $R^5$ is $SO_2Cl$ can be prepared from polystyrene by reaction with chlorosulphonic acid and thionyl chloride in a suitable organic solvent such as dichloromethane.

° Compounds of structure (IV) in which n is 1, X is O or S and M is $NR^1R^2$ can be prepared from compounds of structure (IIA) in which $R^5$ is $CH_2Cl$ (chloromethylated polystyrene, commercially available), by reaction with, for example, a compound of structure $HX(CH_2)_mN(CH_3)_2$ in which X is O or S.

° Compounds of structure (IV) in which n is O, X is CONH and M is $NR^1R^2$ can be prepared from compounds of structure (IIA) in which $R^5$ is COCl, by reaction with a compound of structure $NH_2(CH_2)_mNR^1R^2$. Compounds of structure (IIA) in which $R^5$ is COCl can be prepared from compounds of structure IIA in which $R^5$ is $CO_2H$ by reaction with thionylchloride; and compounds of structure (IIA) in which $R^5$ is $CO_2H$ can be prepared from polystyrene by reaction with $CH_3COCl$ in the presence of $AlCl_3$ (to form compounds (IIA) in which $R^5$ is $COCH_3$), followed by KOBr.

Other possible routes will be apparent to those skilled in the art, and include synthesis of particular intermediates starting from the monomers rather than polystyrene. For example, compounds of structure (IV) in which n is 1, X is O and M is $NR^1R^2$ can be prepared by reaction of styrene, divinylbenzene and a compound of structure (V)

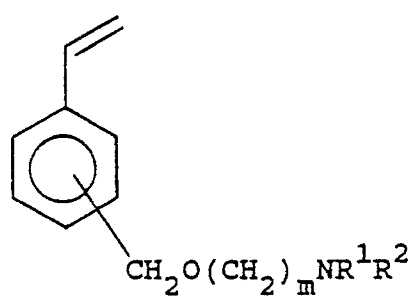

(V)

in which m, $R^1$ and $R^2$ are as described for structure (IV), under standard polymerisation conditions. For example, polymerisation can be carried out in an aqueous suspension, comprising, for example, polyvinyl alcohol in the presence of an initiator at elevated temperature. Suitable initiators include, for example, AIBN.

Compounds of structure (V) can be prepared from the known monomer, chloromethylvinylbenzene using standard procedures.

The polystyrene polymers of structure (I) have been found to bind bile acids in _in vitro_ experiments and in _in vivo_ animal models they have been found to increase the amount of bile acids detectable in the faeces. In particular, when compared to the known sequestrants e.g. cholestyramine, the polymers of structure (I) have surprisingly been found to have an unexpected profile of activity which is thought will provide advantages over the known compounds in the lowering of serum cholesterol levels in animals, in particular humans. More specifically, in _in vitro_ experiments, when compared to cholestyramine the compounds of structure (I) have been found to bind comparable amounts of bile acid per gram of polymer (at physiological concentrations of bile acids), and to bind the bile acid more strongly i.e., the bile acids have been found to dissociate more slowly from the compounds of the invention. It is expected that compounds having such qualities will be able to achieve significant lowering of plasma cholesterol levels at much lower dosages than has hitherto been possible with known sequestrants (currently given at up to 36 g/day).

As indicated earlier it is recognised that removal of bile acids from the intestinal tract in this way lowers serum cholesterol levels and also has a beneficial effect on protecting against atherosclerosis and its dependent clinical conditions. The present invention therefore provides in a further aspect, polystyrene polymers of structure (I) for use in therapy, in particular for the lowering of serum cholesterol levels in mammals, including humans. In addition the polymers of structure (I) are expected to be of use in protecting against atherosclerosis and its sequelae, and for example in the treatment of pruritus and diarrhoea.

In view of the foregoing the present invention also provides a method of lowering serum cholesterol levels in mammals which comprises administering to a mammal in need thereof an effective serum cholesterol lowering amount of a polystyrene polymer of structure (I); and a method of protecting against atherosclerosis.

When used in therapy in the methods of the invention, the polystyrene polymers of structure (I) are in general administered in a pharmaceutical composition.

In a still further aspect of the present invention there is therefore provided a pharmaceutical composition comprising a polystyrene polymer of structure (I) in association with a pharmaceutically acceptable carrier.

The compositions of the present invention can be prepared by techniques well known to those skilled in the art of pharmacy and include all those known for the formulation of polystyrene polymers for human use.

The polymers are preferably administered as formulations in admixture with one or more conventional pharmaceutical excipients which are physically and chemically compatible with the polymer, which are non-toxic, are without deleterious side-effects but which confer appropriate properties on the dosage form.

In general, for liquid formulations, aqueous based pharmaceutically acceptable carriers such as water itself or aqueous dilute ethanol, propylene glycol, polyethylene glycol or glycerol or sorbitol solutions are preferred.

Such formulations can also include preservatives and flavouring and sweetening agents such as sucrose, fructose, invert sugar, cocoa, citric acid, ascorbic acid, fruit juices etc. In general, digestible oil or fat based carriers should be avoided or minimised as they contribute to the condition sought to be alleviated by use of the polymers. They are also subject to absorption by the polymers during prolonged contact, thus reducing the capacity of the polymer to absorb bile acids after administration.

The polymers can also be prepared as 'concentrates', for dilution prior to administration, and as formulations suitable for direct oral administration. They can be administered orally _ad libitum_, on a relatively continuous basis for example by dispersing the polymer in drinks or food.

Preferably, the polymers are administered in tablet form or in gelatin capsules containing solid particulate polymer or a non-aqueous suspension of solid polymer containing a suitable suspending agent. Suitable excipients for such formulations will be apparent to those skilled in the art and include, for example, for tablets and capsules lactose, microcrystalline cellulose, magnesium stearate, povidone, sodium starch glycollate and starches; and for suspensions in capsules, polyethylene glycol, propylene glycol and colloidal silicon dioxide.

Preferably the polymer is administered in unit dosage form, each dosage unit containing preferably from 0.5 g to 1.5 g of polymer.

The daily dosage regimen for an adult patient may be, for example, a total daily oral dose of between 1 and 10 g, preferably 1-5 g, the compound being administered 1 to 4 times a day depending on the size of individual dosage units. Suitably the compound is administered for a period of continuous therapy of one month or more sufficient to achieve the required reduction in serum cholesterol levels.

In addition the polymers of the present invention can be co-administered (together or sequentially) with further active ingredients such as HMGCoA reductase inhibitors and other hypocholesterolaemic agents, and other drugs for the treatment of cardiovascular diseases.

The following data and examples indicate the properties and preparation of the polymers of the present invention. Temperatures are recorded in degrees celsius. The exchange capacity of the ammonium substituted polymers was determined by elemental analysis and/or potentiometric titration of chloride ion. Figures quoted are expressed as milliequivalents of exchangeable chloride ion per gram of dry polymer weight.

## Example 1

(a) Sodium hydride (20 g of a 50% dispersion in oil) was washed with petroleum spirit and suspended in dry dimethylformamide (DMF) (500 ml). 3-(N,N-dimethylamino)propan-1-ol (31 g) was added over 1.5

hours and the reaction stirred under nitrogen at room temperature for 3 hours. 3(4)-Vinylbenzylchloride (50 g) was added to the cooled reaction mixture keeping the temperature at about 30°. The reaction became very thick and frothy so more dry dimethylformamide (500 ml) was added to aid stirring. The reaction was stirred at room temperature for 1 hour. Water was carefully added to the reaction before it was evaporated to dryness. The resulting oil was dissolved in chloroform (500 ml) and washed with water, dried with anhydrous magnesium sulphate and evaporated to dryness. The resulting oil was subjected to silica gel column chromatography (gradient $CHCl_3$:MeOH as eluent) to give 3(4)-[3-(dimethylamino)-propyloxymethyl]-1-vinylbenzene] as an orange oil (41.7 g, 58%).

(b) The above substituted styrene (11.96 g), styrene (4.74 g), divinylbenzene (0.24 g) and azobisisobutyronitrile (AIBN) (0.20 g) were mixed to give a solution and added to a solution of polyvinyl alcohol (mw 125,000) (0.5 g) in distilled water (200 ml). The mixture was then stirred at 80° under an atmosphere of nitrogen at such a rate as to maintain the monomers in suspension. After 6.5 hours, the stirrer was stopped and the mixture poured into distilled water. The polymer formed was washed by decantation with water and acetone, filtered off and dried to give 1% (w/w) cross-linked 3-(dimethylamino)-propyloxymethyl-substituted polystyrene as polymer beads (7.48 g) (3.07 meq N/g).

(c) The above polymer (3.5 g) was suspended in methanol (40 ml), methyl iodide (5 ml) was added and the reaction mixture heated at 65° for 24 hours. The polymer was washed by decantation with methanol, water, aqueous 2N HCl, water, methanol, and ether, filtered off and dried to give 3-(trimethylammonio)-propyloxymethyl-substituted polystyrene, chloride salt, as polymer beads (3.0 g) (2.53 meq $Cl^-$ g).

## Example 2

The polymer prepared in Example 1b (3.5 g) was suspended in DMF (40 ml), hexyl bromide (5.38 g) was added and the reaction mixture heated at 65° for 24 hours. Work-up as for Example 1c gave 3-(N-hexyl-N,N-dimethylammonio)propyloxymethyl-substituted polystyrene, chloride salt, as white polymer beads (3.12 g) (2.22 meq $Cl^-$ g).

## Example 3

3-(N,N-dimethyl-N-octylammonio)propyloxymethyl-substituted polystyrene, chloride salt (4.53 g) (2.02 g meq $Cl^-$/g) was prepared from 3-(dimethylamino)propyloxymethyl-substituted polystyrene (3.5 g) (Example 1b) and octyl bromide (6.2 g) by the method described in Example 1c.

## Example 4

(a) A solution of ll-bromoundecan-1-ol (10 g) and dimethylamine (40 ml, 33% solution in IMS) in ethanol (90 ml) was heated at reflux temperature for 18 hours, then evaporated to dryness. The residue was dissolved in diethyl ether and washed with 5N aqueous sodium hydroxide solution. The ether extracts were combined, filtered, dried and evaporated to give 11- N,N-dimethylamino)undecan-1-ol as a straw-coloured, waxy solid (8.06 g).

(b) 11-(N,N-dimethylamino)undecan-1-ol (78 g) was added under nitrogen to a suspension of sodium hydride (20 g, 50% in oil, washed with petroleum spirit) in DMF (500 ml, distilled), and the mixture stirred at 60° for 4 hours. The 3(4)-vinyl benzyl chloride (50 g) was added keeping the mixture below 39°. The thick red solution so formed was stirred at room temperature for 3.5 hours before treating with methanol. The solvent was removed under reduced pressure and water (600 ml) and chloroform (600 ml) added. The organic layer was separated and rewashed with water (600 ml) before being evaporated to dryness. The residual oil was dissolved in ethyl acetate (600 ml) and washed with water (500 ml), then extracted with 10% aqueous hydrochloric acid (500 ml). The acidic extract was separated, basified with 2N NaOH to pH8 and extracted with ether (500 ml). The ether extract was dried ($MgSO_4$) and the solvent removed to give 3-(4)-(11- N,N-dimethylamino)undecyloxymethyl)styrene as a yellow-brown oil (69.4 g).

(c) This substituted styrene (38.24 g), styrene (11.26 g), divinyl benzene (0.52 g, purity 55%) and AIBN (0.5 g) were mixed to give a homogenous solution and added to a solution of polyvinylalcohol (mw 125,000)

(1.0 g) in distilled water (500 ml). The mixture was stirred at 80° under an atmosphere of nitrogen at such a rate as to maintain the monomers in suspension. After 6 hours the mixture was poured into distilled water. The polymer formed was washed with cold water, hot water, methanol and ether and dried at 60° in vacuo to give 1% (w/w) cross-linked (11-(N,N-dimethylamino)undecyl)oxymethyl-substituted polystyrene as polymer beads (30.2 g) (2.25 meq N/g).

(d) This polymer (6.0 g) was stirred in tetramethylene sulphone (120 ml) with iodomethane (20 ml) at room temperature for 24 hours. The polymer was filtered off, washed with methanol, acetone/hydrochloric acid, and ether. It was then dried at 60° in vacuo for 24 hours, to give [11-(N,N,N-trimethylamino)-undecyloxymethyl-substituted polystyrene, chloride salt, as polymer beads (6.31 g) (2.19 meq $Cl^-$/g).

## Example 5

(a) 2% (w/w) cross-linked 11-(N,N-dimethylamino)undecyloxymethyl-substituted polystyrene was prepared by co-polymerising 11-(N,N-dimethylamino)undecyloxymethylstyrene (38.27 g) (Example 4b), styrene (10.01 g) and divinylbenzene (1.80 g, purity 55%) as described in Example 4c. After work-up the polymer was isolated as polymer beads (34.9 g) (2.26 meq N/g).

(b) The above polymer (6.0 g) was reacted with iodomethane (20 ml) under the conditions described in Example 4d to give, after work-up, (11-(trimethylamino)undecyl)oxymethyl-substituted polystyrene, chloride salt, as polymer beads (6.09 g) (2.03 meq $Cl^-$/g).

## Example 6

(a) Sodium hydride (10.5 g, 50% dispersion in oil) was washed with hexane and suspended in DMF (250 ml) under nitrogen. To this suspension a solution of 3(4)-vinylbenzylthiol (29.8 g) in DMF (100 ml) was added and the mixture stirred at 50° for 1 hour, then cooled to 10°. 1,10-Dibromodecane (150 g) in dry tetrahydrofuran (THF) (400 ml) was then added during 2.5 hours, and the subsequent mixture allowed to stir at room temperature for 60 hours. The mixture was then evaporated to dryness and the residue dissolved in diethyl-ether (500 ml), washed with distilled water (2 x 200 ml), dried and evaporated to give a brown oil. This oil was subjected to silica gel chromatography to give 3(4)-((10-bromodecyl)thiomethyl)styrene (12.4 g) as a pale yellow oil.

(b) The above substituted thiomethylstyrene (10 g), styrene (4.71 g), divinylbenzene (0.292g, 55% purity) and AIBN (0.2 g) were mixed to give a solution and added to a solution of polyvinylalcohol (mw 125,000) (0.5 g) in water (250 ml). This mixture was then stirred at 80° as described in Example 1b for 6.5 hours. The resulting polymer bead were washed with water, acetone, ether, methanol, and acetone then dried at room temperature to give 1% (w/w) cross-linked 10-bromodecylthiomethyl-substituted polystyrene as polymer beads (10.53 g) (2.39 meq S/g).

(c) A suspension of the above polymer (8 g) in DMF (100 ml) was treated with trimethylamine in industrial methylated spirits (IMS) (100 ml, 33% solution). This mixture was stirred at 60° for 24 hours, with the addition of a further portion of trimethylamine solution (50 ml) after 6 hours. The polymer was then filtered off and washed with methanol, aqueous methanolic hydrochloric acid (1N HCl), aqueous 2N HCl, water, methanol and diethyl ether, and dried at 80° for 20 hours to give (10-(trimethylamino)decyl)-thiomethyl-substituted polystyrene, chloride salt, as polymer beads (2.10 meq $Cl^-$/g, 2.0 meq N/g). This polymer was sieved and the major fraction (6.18 g) (106-212 μ) used.

## Example 7

(a) A suspension of 3(4)-((10-bromodecyl)thiomethyl)styrene (Example 6a) (15.58 g) in methanol (200 ml) was treated with a solution of potassium hydrogen persulphate (oxone) (77.9 g) in water (150 ml) at 50°. The resulting mixture was stirred at room temperature for 4 hours, water (1000 ml) added and the aqueous mixture extracted with chloroform (4 x 200 ml). The chloroform extracts were combined, washed with water, dried and evaporated. The residual oil was chromatographed on silica gel to give 3(4)-((10-bromodecyl)-S,S-dioxothiomethyl)styrene as a colourless solid which was recrystallised from ethanol/water,

m.p. 74-76° (10.14 g).

(b) A mixture of the above substituted styrene (10 g), styrene (4.73 g), divinylbenzene (0.27 g, 55% purity) and AIBN (0.2 g) was treated as described in Example 6b to give 2% (w/w) cross-linked (10-bromodecyl)-S,S-dioxothiomethyl-substituted polystyrene as polymer beads (10.81 g) (1.70 meq Br/g).

(c) A suspension of the above substituted polystyrene (8 g) in DMF (100 ml) was treated with trimethylamine in IMS (100 ml), 33% solution) at 70° for 26 hours. After work-up as described in Example 6c the polymer was dried at 80° in vacuo for 20 hours to give 10-(N,N,N-trimethylammonio)decyl-S,S-dioxothiomethyl-substituted polystyrene, chloride salt, as polymer beads (8.18 g) (1.43 meq $Cl^-$/g).

## Example 8

(a) A suspension of chloromethylated polystyrene (40.2 g) (3.72 meq Cl/g) in DMF (400 ml) was treated with potassium phthalimide (33.2 g) and the mixture heated at 90° for 19 hours. The polymer was filtered off and washed with ethanol, water, ethanol and diethyl ether and dried. This polymer (56.3 g) was then suspended in IMS (500 ml) and treated with hydrazine hydrate (100 ml) at reflux temperature for 10 hours. 4N aqueous sodium hydroxide solution (400 ml) was added and the insoluble polymer filtered off and washed with 4N sodium hydroxide solution, water, ethanol, and diethyl ether. After drying at 90° aminomethyl-substituted polystyrene (3.69 meq N/g) was isolated as polymer beads (36.5 g).

(b) A suspension of the above aminomethyl-substituted polystyrene (10 g) and sodium carbonate (30 g) in dry tetrahydrofuran (THF) (200 ml) was treated with 6-bromohexanoyl chloride (23.5 g) at room temperature for 12 hours. The polymer was then filtered off and washed with water, aqueous 2N HCl, water, methanol and diethyl ether and dried to give (6-bromohexanamido)-methyl-substituted polystyrene as polymer beads (16.8 g) (2.13 meq Br/g).

(c) A suspension of the above polymer (3.0 g) in DMF (15 ml) was treated with trimethylamine in IMS (25 ml, 33% solution) at room temperature for 7 days. The polymer was then filtered off and washed with aqueous 2N NaOH, aqueous 2N HCl, water, methanol and ether and dried to give 6-(N,N,N-trimethylammonio)hexanamidomethyl- substituted polystyrene, chloride salt, as polymer beads (2.97g) (2.11 meq $Cl^-$/g).

## Example 9

A suspension of the (6-bromohexanamido)methyl-substituted polystyrene (3.0 g), prepared in Example 8b, in DMF (100 ml) was treated with N,N-dimethyloctylamine (7.87 g) at 63° for 72 hours. The polymer was filtered off, washed as described in Example 8c and dried to give 6-(N,N-dimethyl-N-octylammonio)-hexanamidomethyl-substituted polystyrene, chloride salt, as off-white polymer beads (3.6 g) (1.80 meq $Cl^-$/g, 3.59 meq N/g).

## Example 10

A suspension of the (6-bromohexanamido)methyl-substituted polystyrene (5.0 g), prepared in Example 8b, in pyridine (50 ml) was heated at reflux temperature for 18 hours. The polymer was filtered off, washed as described in Example 8c and dried to give 6-(1-pyridine)hexanamidomethyl-substituted polystyrene, chloride salt, as off-white polymer beads (5.59 g) (1.98 meq $Cl^-$/g, 3.98 meq N/g).

## Example 11

(a) Aminomethyl-substituted polystyrene (Example 8a) (10 g) was treated with 11-bromoundecanoyl chloride (50 g) and sodium carbonate (25 g) in THF (250 ml) at room temperature for 18 hours. The polymer was filtered off and treated with further quantitites of 11-bromoundecanoyl chloride (30 g) and sodium carbonate (25 g) in THF (250 ml) for 72 hours. The polymer was filtered off and washed with water,

IMS and diethyl ether and dried to give 11-bromoundecanamidomethyl-substituted polystyrene as off-white polymer beads (19.6 g) (1.63 meq Br/g).

(b) The above polymer (4 g) was suspended in a solution of trimethylamine in IMS (100 ml, 33% solution) and stirred at 50° for 18 hours. The polymer was filtered off and washed as described in Example 8c to give 11-(N,N,N-trimethylammonio)undecanamidomethyl-substituted polystyrene, chloride salt, as off-white polymer beads (4.86 g) (1.52 meq $Cl^-$/g, 3.33 meq N/g).

## Example 12

A suspension of 11-bromoundecanamidomethyl-substituted polystyrene (Example 11a) (5 g) in DMF (100 ml) was treated with N,N-dimethyloctylamine (15.8 g) and the mixture stirred at 60° for 24 hours. The polymer was filtered off and washed as in Example 8c to give 11-(N,N-dimethyl-N-octylammonio)-undecanamidomethyl-substituted polystyrene, chloride salt, as off-white polymer beads (5.38 g) (1.34 meq $Cl^-$/g).

## Example 13

A suspension of 11-bromoundecanamidomethyl-substituted polystyrene (Example 11a) (5 g), in pyridine (50 ml) was heated at 60° for 18 hours then the polymer filtered off and washed as in Example 8c to give 11-(1-pyridinio)undecanamidomethyl-substituted polystyrene, chloride salt, as off-white polymer beads (5.16 g) (1.55 meq $Cl^-$ g, 3.17 meq N/g).

## Example 14

(a) 1% cross-linked polystyrene beads (divinylbenzene/styrene co-polymer) were nitrated by the method of Hodge et al (Polymer, 1985, 26, 1701) and the nitrated polystyrene reduced by the method of Dowling and Stark (Biochemistry, 1969, 8, 4728) to give amino-substituted polystyrene (4.59 meq N/g) polymer.

(b) A suspension of the above amino-substituted polystyrene (5 g) in dry dichloromethane (40 ml) was treated with a solution of 11-bromoundecanoyl chloride (9.36 g) (Example 11a) in dry dichloromethane (15 ml) at -20 to -10° during 10 minutes. A solution of N,N-dimethyldodecylamine (9.4 g) in dichloromethane (10 ml) was then added and the mixture warmed to room temperature and stirred for 20 hours. The polymer was then filtered off, washed with dichloromethane, methanol, THF, water, THF and methanol, then air dried. This polymer was suspended in DMF (40 ml), N,N-dimethyldodecylamine (14.1 g) added and the mixture heated at 65° for 24 hours. The polymer was filtered off, washed as described in Example 1c and dried to give [11-(N,N-dimethyl-N-dodecylammonio)undecanamido]-substituted polystyrene, chloride salt, as polymer beads (12.40 g) (1.62 meq $Cl^-$ g, 3.10 meq N/g).

## Example 15

(a) Amino-substituted polystyrene (5.28 meq N/g) was prepared as described in Example 14a. This polymer was converted to the N-methylamino-substituted polystyrene by the mono-methylation procedure of Krishnamurthy (Tet. Lett., 1982, 23, 3315) giving N-methylamino-substituted polystyrene which contained 4.77 meq N/g polymer.

(b) This polymer (2.4 g) was treated with bromoundecanoyl chloride (5.41 g) and N,N-dimethyldodecylamine (5.42 g) in dichloromethane, followed by treatment with N,N-dimethyldodecylamine (8.13 g) in DMF in a manner analogous to that described in Example 14b to give N-methyl-11-(N,N-dimethyl-N-dodecylammonio)undecanamido-substituted polystyrene (6.03 g (1.62 meq $Cl^-$/g, 3.06 meq N/g).

Example A

A liquid formulation for oral administration is prepared from the following:

| | (w:v) |
|---|---|
| Compound of Structure (I) | 10% |
| Avicel RC591 | 1.25% |
| Antifoam emulsion | 0.05% |
| Flavours | 0.02% |
| Sodium saccharide | 0.01% |
| Preservatives : | |
| Methyl Parabenz | 0.12% |
| Propyl Parabenz | 0.04% |
| Sorbitol syrup (70%) | 30% |
| Glycerin | 5% |
| Water | to 100% |

Example B

A capsule formulation for oral administration is prepared by incorporating the following into a soft gelatin capsule :
Compound of Structure (I) (500 mg), Aerosil 200 (5 mg), Magnesium Stearate (5 mg), Avicel PH101 (40 mg), and, optionally, sodium starch glycollate (10 mg).

Example C

A food additive formulation for example a sachet for reconstitution or mixing with food, is prepared by incorporating into a powder formulation, compound of Structure (I) (2500 mg), sodium carboxymethylcellulose (50 mg), sucrose (2400 mg) and flavours (50 mg).

DATA

Bile Acid Dissociation assay.

Test compound (150 mg) was equilibrated with 5 mM sodium glycocholate (30 ml) - a typical physiological concentration - in Krebs' buffer for 3 hours. The compound was separated by centrifugation and the total bound determined by subtraction of the amount in the supernatant from the total bile acid used. Bile acid dissociation was measured by resuspending the compound in Krebs' buffer, shaking and sampling the mixture through a filter at several time points up to 20 minutes. Radioactivity and hence bile acid dissociated was determined in the filtrate (Table I).

Table I

| | | GC Bound mmoles/g | |
|---|---|---|---|
| Example No. | t-O | t = 2 minutes | % Dissociated |
| 1 | 0.83 | 0.69 | 17 |
| 2 | 0.79 | 0.61 | 23 |
| 3 | 0.81 | 0.70 | 10 |
| 4 | 0.92 | 0.84 | 9 |
| 5 | 0.91 | 0.84 | 8 |
| 6 | 0.89 | 0.85 | 4 |
| 8 | 0.79 | 0.66 | 16 |
| 9 | 0.74 | 0.61 | 18 |
| 10 | 0.77 | 0.56 | 27 |
| 11 | 0.52 | 0.46 | 12 |
| 12 | 0.46 | 0.39 | 15 |
| 13 | 0.58 | 0.50 | 14 |
| 14 | 0.54 | 0.44 | 19 |
| 15 | 0.75 | 0.61 | 19 |

## Claims

1. A polymer of structure (1)

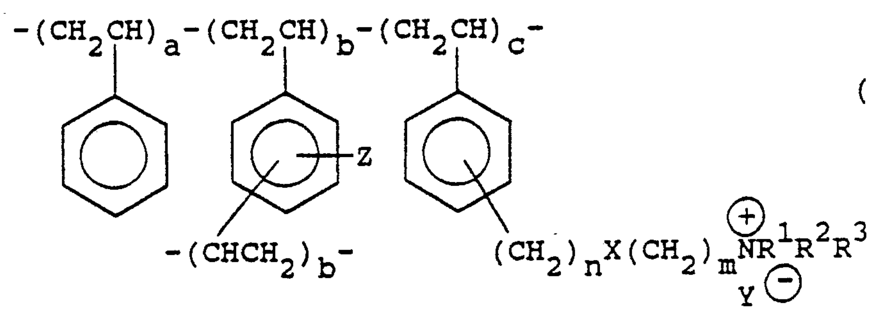

(I)

in which,
n is 0 or 1;
m is 1 to 20;
Z is H or a group $(CH_2)_nX(CH_2)_m\overset{\oplus}{N}R^1R^2R^3Y^\ominus$,
X is O, S, $SO_2$, amido or sulphonamido;
$R^1$ and $R^2$ are the same or different and are each $C_{1-4}$alkyl;
$R^3$ is $C_{1-20}$alkyl; or $R^1$ to $R^3$ together with the nitrogen atom to which they are attached form a ring, optionally containing one or more further heteroatoms and optionally substituted by a $C_{1-4}$alkyl group;
a, b and c are numbers which indicate the relative molar percentages of the units present in a random distribution in said polymer, (b) being from about 0.5 to about 10 molar percent, and (c) being from about 30 to about 99 molar percent; and
$Y^\ominus$ is a physiologically acceptable counter ion.

2. A polystyrene polymer as claimed in claim 1 in which $R^1$, $R^2$ and $R^3$ are each methyl.

3. A polystyrene polymer as claimed in claim 2 in which (b) is from about 1 to about 4 molar percent of said polymer..

4. A polystyrene polymer as claimed in claim 1 which is

[11- N,N,N-trimethylamino)undecyloxymethyl-substituted polystyrene, chloride salt;
(11-(trimethylamino)undecyl)oxymethyl-substituted polystyrene, chloride salt;
(10-(trimethylamino)decyl)thiomethyl-substituted polystyrene, chloride salt.

5. A process for the preparation of a polystyrene polymer as claimed in any one of claims 1 to 4 which comprises

(a) reaction of a polymer of structure (11)

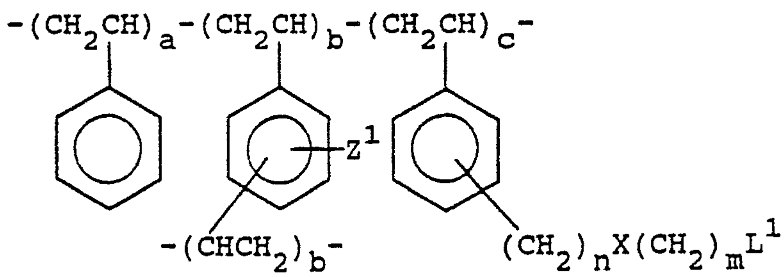

(II)

in which $Z^1$ is hydrogen or $(CH_2)_nX(CH_2)_mL^1$ and X, a, b, c, n and m are as described for structure (I) in claim (I) and $L^1$, is a group displaceable by an amine, with an amine of structure $R^1R^2R^3N$ (III) in which $R^1$ to $R^3$ are as described for structure (I); or

(b) for compounds in which $R^1$ and $R^2$ are the same or different and are each $C_{1-4}$alkyl and $R^3$ is a $C_{1-20}$ alkyl group, reaction of a compound of structure (IV)

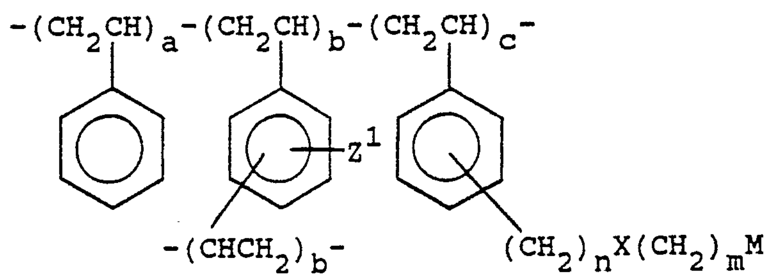

(IV)

in which $Z^1$ is hydrogen or $(CH_2)_nX(CH_2)_mM$, X, a, b, c, n and m are as described for structure (I) in claim 1 and M is $NR^1R^2$ or $NR^2R^3$ in which $R^1$ to $R^3$ are as described for structure (I) in claim 1 with a compound of structure $R^4L^2$ (V) in which $R^4$ is a $C_{1-4}$alkyl group when M is $NR^1R^3$ or a $C_{1-20}$ alkyl group When M is $NR^1R^2$ and $L^2$ is a group displaceable by an amine.

6. A pharmaceutical composition comprising a polystyrene polymer of structure (I) as claimed in claim 1 in association with a pharmaceutically acceptable carrier.

7. A polystyrene polymer as claimed in claim 1 for use as a therapeutic agent.

8. A polystyrene polymer as claimed in claim 1 for use in the lowering of serum cholesterol levels.

9. A polystyrene polymer of structure (II) or (IV).